# EUROPEAN PATENT APPLICATION

(11) **EP 2 180 315 A1**
(43) Date of publication of application: **28.04.2010**
(21) Application number: 08826860.2
(22) Date of filing: 31.07.2008
(51) Int. Cl.: G01N 27/416, G01N 1/10, G01N 27/327

(54) **STRESS MEASUREMENT KIT AND STRESS MEASUREMENT METHOD**

(30) Priority: 01.08.2007 JP 2007201159
(71) Applicant: Nipro Corporation, Kita-ku Osaka-shi, Osaka 531-8510 (JP)
(72) Inventor: MORITA, Mitsuhiro, Osaka-shi Osaka 531-8510 (JP); BABA, Toshiaki, Osaka-shi Osaka 531-8510 (JP); YOSHIDA, Hiroshi, Osaka-shi Osaka 531-8510 (JP); NISHIMURA, Emi, Osaka-shi Osaka 531-8510 (JP)
(74) Representative: Albrecht, Thomas
(86) International application number: PCT/JP2008/063739
(87) International publication number: WO 2009/017188

(57) **Abstract**

It is an object of the present invention to provide a stress measurement kit that is economical and that has good sensitivity. A stress measurement kit for measuring a stress level of a test subject includes: (A) a sensor chip including an electrically insulating substrate, and an electrode system that is placed on the electrically insulating substrate and that includes at least a working electrode and a counter electrode, wherein glucose dehydrogenase (GDH) and an electron mediator are immobilized on the working electrode; (B) a polysaccharide; and (C) a sensor body including an insertion hole into which the sensor chip is inserted, an electrical measurement means that is electrically connected to the electrode system of the sensor chip in a state where the sensor chip is inserted into the insertion hole, and a conversion means by which a current value or an electric quantity measured by the electrical measurement means is converted into an amylase activity value.

## Description

### Technical Field

The present invention relates to a stress measurement kit and a stress measurement method.

### Background Art

A salivary amylase (hereinafter, occasionally abbreviated as "α-Amy") activity has been attracting attention as an indicator that enables stress to be noninvasively measured. For example, Patent Document 1 discloses a reagent in which a modified oligosaccharide and a competitive inhibitor are carried on a nonwoven fabric on a support. This reagent enables a salivary amylase activity to be measured without diluting saliva.

However, since this measurement system is an optical measurement system, a light source and a light-receiving means have to be mounted on an apparatus, and a certain optical path has to be secured therein. Accordingly, there is a limitation in reducing the size of the apparatus. That is to say, although this apparatus is suitable for use in monitoring stress levels indoors, there is a problem in that this apparatus is slightly difficult to use as a portable one.

As the most effective means for solving this problem, it is conceivable to employ an electrode system as a system for measuring an amylase activity. As an electrode system for measuring an amylase activity, a reaction system using α-glucosidase and glucose oxidase as in Scheme 1 is well known as a theoretically practicable system (Patent Documents 2 and 3).

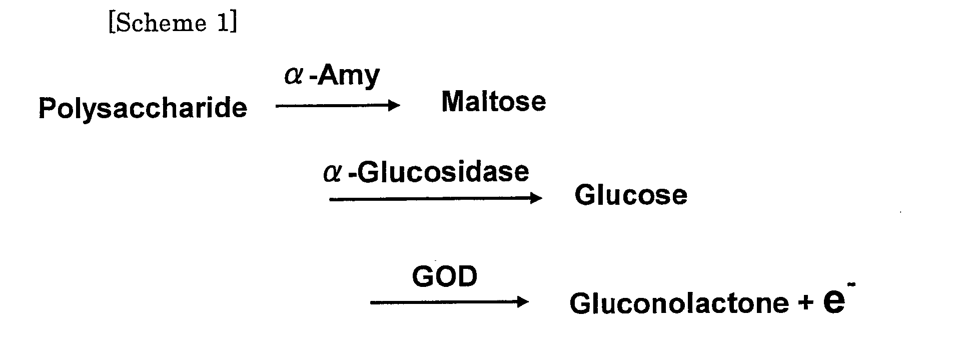

However, in the case where this system is actually applied to the production of an electrode system, the measurement sensitivity to be required cannot be attained. For example, as clearly seen in Fig. 21 of Patent Document 3, the measurement sensitivity is at a nanoampere level, which is not sufficient for a commercially available stress measurement apparatus. The reason for this may be that the second-row reaction or the third-row reaction in Scheme 1 does not occur because either α-glucosidase or glucose oxidase is inactivated. In particular, it seems that α-glucosidase is easily inactivated when immobilized on an electrode. It is also conceivable that excessive amounts of these enzymes are immobilized in order to efficiently cause the reactions, but this leads to another problem in which the material cost of the sensor chip increases.
Patent Document 1: Japanese Laid-Open Patent Publication No. 2004-024236
Patent Document 2: Japanese Laid-Open Patent Publication No. 10-262645
Patent Document 3: Japanese Laid-Open Patent Publication No. 2005-249530

### Brief Description of Drawings

Fig. 1 is a view showing an embodiment of the stress measurement kit of the present invention.
Fig. 2 is a view showing an embodiment of the saliva-collecting tool of the present invention.
Fig. 3 is a graph showing the results of experimental examples.

### Description of Reference Numerals

- 1: Sensor chip
- 11: Electrically insulating substrate
- 121: Working electrode
- 122: Counter electrode
- 2: Sensor body
- 21: Insertion hole
- 211: Terminal for connection to working electrode
- 212: Terminal for connection to counter electrode
- 22: Electrical measurement means
- 23: Conversion means
- 3: Saliva-collecting tool
- 31: Shaft member
- 32: Grip portion
- 33: Villous support
- 34: Polyamide villi

### Disclosure of Invention

### Problems to be Solved by the Invention

It is an object of the present invention to provide a stress measurement kit that is economical and that has good measurement sensitivity.

### Means for Solving the Problems

The present inventions relate to:
[1] a stress measurement kit for measuring a stress level of a test subject, comprising:
   (A) a sensor chip including an electrically insulating substrate, and an electrode system that is placed on the electrically insulating substrate and that includes at least a working electrode and a counter electrode, wherein glucose dehydrogenase (GDH) and an electron mediator are immobilized on the working electrode;
   (B) a polysaccharide; and
   (C) a sensor body including an insertion hole into which the sensor chip is inserted, an electrical measurement means that is electrically connected to the electrode system of the sensor chip in a state where the sensor chip is inserted into the insertion hole, and a conversion means by which a current value or an electric quantity measured by the electrical measurement means is converted into an amylase activity value;
[2] the stress measurement kit described in [1], wherein the polysaccharide is immobilized on the working electrode of the sensor chip;
[3] the stress measurement kit described in [1], wherein a coenzyme of glucose dehydrogenase is further immobilized on the working electrode;
[4] the stress measurement kit described in [3], wherein the coenzyme of glucose dehydrogenase is at least one selected from the group consisting of pyrroloquinoline quinone (PQQ), nicotinamide adenine dinucleotide (NAD), and nicotinamide adenine dinucleotide phosphate (NADP);
[5] the stress measurement kit described in [1], further comprising a saliva-collecting tool;
[6] the stress measurement kit described in [5], wherein the saliva-collecting tool includes a shaft member, a grip portion that is provided at one end of the shaft member, a villous support that is provided at another end of the shaft member, and polyamide villi that are supported by the villous support;
[7] a stress measurement method for measuring a stress level of a test subject using a stress measurement kit,
   wherein the stress measurement kit comprises:
   (A) a sensor chip including an electrically insulating substrate, and an electrode system that is placed on the electrically insulating substrate and that includes at least a working electrode and a counter electrode, wherein glucose dehydrogenase (GDH) and an electron mediator are immobilized on the working electrode;
   (B) a polysaccharide; and
   (C) a sensor body including an insertion hole into which the sensor chip is inserted, an electrical measurement means that is electrically connected to the electrode system of the sensor chip in a state where the sensor chip is inserted into the insertion hole, and a conversion means by which a current value or an electric quantity measured by the electrical measurement means is converted into an amylase activity value or a stress level; and
      the stress measurement method comprises the steps of:
      (1) allowing the polysaccharide to act on collected saliva;
      (2) developing the saliva, on which the polysaccharide has acted, in the electrode system;
      (3) measuring a current generated in the electrode system, as a current value or an electric quantity, using the electrical measurement means; and
      (4) converting the current value or the electric quantity measured by the electrical measurement means, into an amylase activity value or a stress level, using the conversion means;
[8] the stress measurement method described in [7],
   wherein the polysaccharide is immobilized on the working electrode of the sensor chip, and
   the steps (1) and (2) are simultaneously performed.
[9] the stress measurement method described in [8], wherein the step (1) is performed by transferring saliva collected by a saliva-collecting tool, to the sensor chip; and
[10] the stress measurement method described in [9], wherein the saliva-collecting tool includes a shaft member, a grip portion that is provided at one end of the shaft member, a villous support that is provided at another end of the shaft member, and polyamide villi that are supported by the villous support.

### Effect of the Invention

The present invention can provide an apparatus that can be more economically produced and that has better measurement sensitivity, than conventional stress measurement apparatuses that use an electrochemical method.

### Best Mode for Carrying Out the Invention

The stress measurement kit of the present invention refers to a kit for electrochemically measuring an amylase activity value and/or a stress level of a test subject, in saliva of the test subject. The test subject is not limited to humans, and may include pets cared for in everyday life, such as dogs, cats, rabbits, and hamsters, and farm animals, such as pigs, horses, sheep, and cattle.

Electrochemical reactions in the present invention refer to the reactions shown in Scheme 2 below.

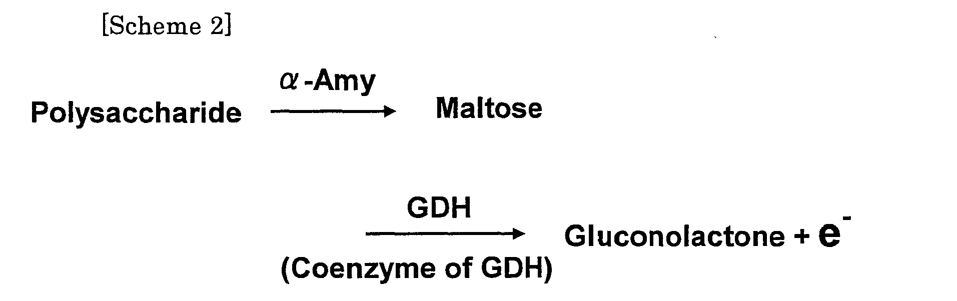

Gluconolactone in Scheme 2 above is formally referred to as 4-O-(α-D-glucopyranoside)-D-glucono-1,5-lactate.

The stress measurement kit of the present invention includes a sensor chip 1, a polysaccharide, and a sensor body 2. Hereinafter, each constituent element will be described with reference to the drawings.

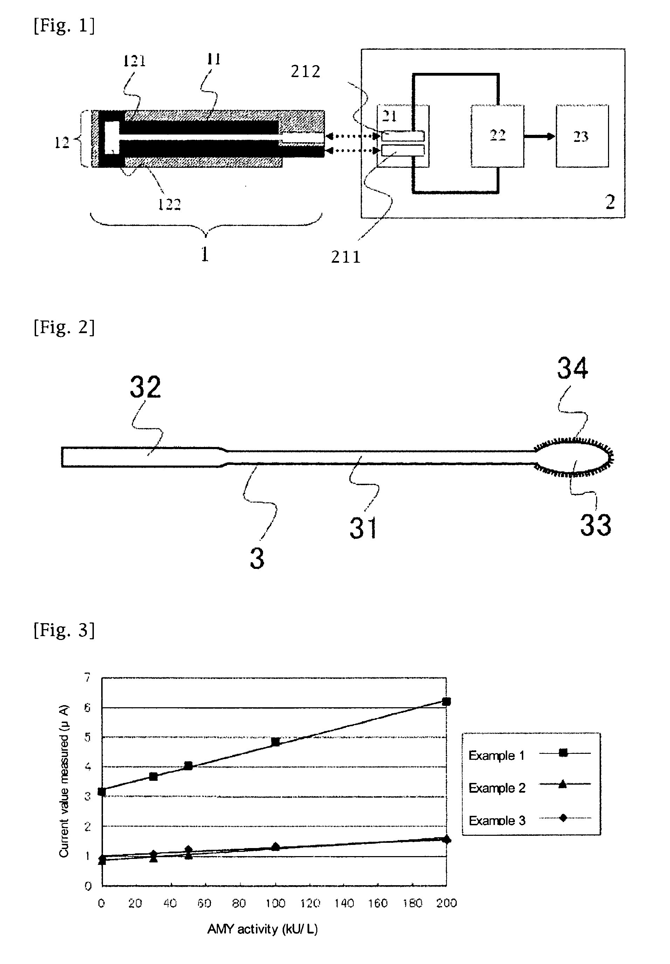

Fig. 1 is a view showing an embodiment of the sensor chip 1 of the present invention. In the present invention, the sensor chip refers to a device that causes an electrochemical reaction with amylase in collected saliva, and guides a current generated by the electrical reactions into the sensor body 2 (described later). The sensor chip is configured so as to include an electrically insulating substrate 11 and an electrode system that is placed on the electrically insulating substrate 11 and that includes at least a working electrode 121 and a counter electrode 122. Glucose dehydrogenase (GDH) and an electron mediator are immobilized on the working electrode 121.

The electrically insulating substrate 11 forms a base for the sensor chip 1 and is a substrate made of an electrically insulating material. Examples of the material include polyester, fluorin-contained resin, and polycarbonate. Polyester is particularly preferable because the material cost is low, but the present invention is not limited to these.

The electrode system refers to a system through which a current generated by the electrochemical reactions on the electrode system flows, and that can be electrically connected to the sensor body 2 (described later). The electrode system forms an electric circuit with the sensor body 2 (described later) in the presence of saliva. The electrode system is configured so as to include at least the working electrode 121 and the counter electrode 122. Accordingly, the electrode system may further include, for example, additional electrodes, such as a reference electrode for confirming the presence of saliva, and an electrode for confirming insertion of the sensor chip 1 into the sensor body 2 (described later).

The working electrode 121 refers to an electrode on which reagents for causing the electrochemical reactions are immbilized and through which a current generated by the electrochemical reactions flows. The working electrode 121 is made of a conductive material. There is no particular limitation on the conductive material, and examples thereof include gold, carbon, platinum, ruthenium oxide, and palladium. The working electrode 121 is formed using a method such as vapor deposition or painting.

The counter electrode 122 refers to an electrode that is placed on the electrically insulating substrate 11 so as to be spaced away from the working electrode 121, and that forms an electric circuit with the working electrode 121 and the sensor body 2 (described later) in the presence of saliva. The counter electrode 122 is made of a conductive material. As in the case of the working electrode 121, there is no particular limitation on the conductive material, and examples thereof include gold, carbon, platinum, ruthenium oxide, and palladium. Furthermore, as in the case of the working electrode 121, the counter electrode 122 is formed using a method such as vapor deposition or painting.

Furthermore, the working electrode 121 and the counter electrode 122 in the electrode system may be arranged so as to oppose each other, or may be arranged on the same surface. There is no particular limitation on the arrangement.

Glucose dehydrogenase (GDH) and an electron mediator are immobilized on the working electrode 121. Glucose dehydrogenase (GDH) and an electron mediator need only be immobilized on the working electrode 121 to such an extent that is sufficient to prevent them from flowing away from the working electrode 121. For example, they may be immobilized through a chemical bond, such as covalent bond, ionic bond, coordinate bond, and Van der Waals bond, or through film formation on the working electrode 121, such as coating.

Glucose dehydrogenase (GDH) refers to an enzyme that decomposes maltose into gluconolactone(4-O-(α-D-glucopyranoside)-D-glucono-1,5-lactate) and that causes reduction through electron donation. Glucose dehydrogenase (GDH) may be used alone, but is preferably used in combination with a coenzyme in order to improve the reaction efficiency. Typical examples of coenzymes of glucose dehydrogenase (GDH) include pyrroloquinoline quinone (PQQ), nicotinamide adenine dinucleotide (NAD), and nicotinamide adenine dinucleotide phosphate (NADP). The electrochemical reactions in the present invention occur in a single step, and, thus, the enzyme does not have to be immobilized on the working electrode 121 more than necessary. Accordingly, an economical sensor chip 1 can be provided. Furthermore, high measurement sensitivity can be stably attained.

Furthermore, the electron mediator refers to a compound that acts so as to mediate electron transfer between collected saliva and the working electrode 121. There is no particular limitation on the electron mediator as long as it is a compound containing a transition metal, such as ruthenium, osmium, iron, and cobalt. Examples of the electron mediator include potassium hexacyanoferrate(II), potassium hexacyanoferrate(III), ferrocene, 1,1'-dimethylferrocene, ferrocenecarboxylic acid, ferrocenecarboxaldehyde, hydroquinone, bromanil, octacyanotungstate, and octacyanomolybdenum. The above-described electrode system can be formed by mixing such an electron mediator with a metal that forms the electrode system, and then subjecting the resulting substance to a method, such as vapor deposition or painting.

Furthermore, the electron mediator may contain a polymer. Examples thereof include (i) poly(vinylferrocene), (ii) ionic polymers, such as polycation-hexacyanoferrate(II) salt and polycation-hexacyanoferrate(III) salt, and (iii) organic transition metal complex polymers, such as a polymer in which an osmium-(2,2-dipyridyl) complex is coordinated to poly(4-vinylpyridine).

Furthermore, a preferable example of an electron mediator containing the above-described polymer includes an organic transition metal complex polymer that contains osmium. Hereinafter, in this specification, such a polymer is referred to as an osmium complex-containing polymer. Examples of the osmium complex-containing polymer include a compound in which an osmium complex such as an osmium-(2,2'-bipyridine) complex or an osmium-(1,10-phenanthroline) complex is coordinated to a polymer such as poly(1-vinylimidazole) or poly(4-vinylpyridine).

In addition, a preferable example of the osmium complex-containing polymer is a coordination compound in which an osmium-(2,2'-bipyridine) complex is coordinated to poly(1-vinylimidazole), in order to enable glucose dehydrogenase (GDH) to be easily immobilized on the working electrode 121. The reason for this is that this osmium complex-containing polymer can be crosslinked with glucose dehydrogenase (GDH) using a crosslinking agent, such as polyethylene glycol diglycidyl ether.

The stress measurement kit of the present invention further contains a polysaccharide. Polysaccharides in the present invention refer to sugar-chain polymers in which glucose is a monomer unit, and are compounds that are degraded by salivary amylase and that are not degraded by glucose dehydrogenase (GDH) and a coenzyme of glucose dehydrogenase (GDH). Examples thereof include dextrin, glycogen, cornstarch, and soluble starch.

The embodiment of the polysaccharide contained in the stress measurement kit of the present invention may be an embodiment in which the polysaccharide is contained in the sensor chip 1, an embodiment in which the polysaccharide is contained in the sensor body 2 (described later), or an embodiment in which the polysaccharide is contained independently from the sensor chip 1 and the sensor body 2. Examples thereof include an embodiment in which the polysaccharide is immobilized on the working electrode 121, and an embodiment in which a conjugate pad impregnated with the polysaccharide is disposed in a flow path that guides saliva, from the mouth from which saliva is collected, to the sensor chip 1. Another example is an embodiment in which the sensor chip 1 or the sensor body 2 (described later) is provided with a reagent chamber that contains a polysaccharide, and in which a flow path that guides the polysaccharide from the reagent chamber to the electrode system is provided in the sensor chip 1 and/or the sensor body 2 (described later). Further, an embodiment in which absorbent cotton or the like of a saliva-collecting tool 3 (described later) is impregnated with the polysaccharide may be adopted.

The embodiments described in the preceding paragraph can be selected in view of the viscosity of the polysaccharide, the solubility of the polysaccharide in water, the molecular weight of the polysaccharide, and other properties. For example, in the case where the polysaccharide is soluble in water, and have a large molecular weight, the polysaccharide can easily form a film, and, thus, the embodiment in which the polysaccharide is immobilized on the working electrode 121 can be selected. This embodiment is preferable because the sensor chip 1 does not have to be provided with elements such as a conjugate pad, a flow path, and a reagent chamber, which can keep the production cost low. Examples of such a polysaccharide include cornstarch and soluble starch.

On the other hand, there is no particular limitation on the sensor body 2 as long as it is a machine that can measure the value of current or the quantity of electricity generated in the sensor chip 1. This sensor body 2 is provided with at least an insertion hole 21, an electrical measurement means 22, and a conversion means 23.

There is no particular limitation on the insertion hole 21 as long as the sensor chip 1 can be inserted thereinto. The insertion hole 21 is provided with terminals 211 and 212 that are electrically connected to the working electrode 121 and the counter electrode 122 of the sensor chip 1, respectively. In the case where the sensor chip 1 is provided with additional electrodes, such as a reference electrode for confirming the presence of saliva, or an electrode for confirming insertion of the sensor chip 1 into the sensor body 2, as described above, terminals that are brought into electrical contact with these electrodes will be further provided.

The electrical measurement means 22 refers to a means that is electrically connected to the terminals arranged in the insertion hole 21, and that measures the value of current or the quantity of electricity generated through the electrochemical reactions in the sensor chip 1. Examples of the electrical measurement means 22 include a coulometer, a potentiometer, and an amperometer.

There is no particular limitation on the conversion means 23 as long as it is a medium that stores a program for converting the current value or the electric quantity measured by the electrical measurement means 22 into an amylase activity value. In connection with the method for converting the current value or the electric quantity into an amylase activity value, calculation can be performed using a calibration curve plotted in advance, and, thus, a detailed description thereof will not be provided herein. The results obtained through calculation by the conversion means 23 are displayed on a display means (not shown) provided in the sensor body 2. At that time, an amylase activity value itself may be displayed, or the amylase activity value may be converted into another parameter that is referred to as a stress level and then displayed. Furthermore, the item to be displayed is not limited to numerical values, and characters corresponding to the numerical values may be displayed.

Furthermore, the stress measurement kit of the present invention may further include a saliva-collecting tool 3. That is to say, the saliva-collecting tool 3 is included such that, after saliva is collected by the saliva-collecting tool 3, the collected saliva is developed in an electrode system 12 of the sensor chip 1. Examples of the saliva-collecting tool 3 include (A) a tool as shown in Fig. 2, including a shaft member 31, a grip portion 32 that is provided at one end of the shaft member 31, a villous support 33 that is provided at the other end of the shaft member 31, and polyamide villi 34 that are supported by the villous support, (B) a so-called cotton swab, and (C) a combination of absorbent cotton or the like and a container that accommodates the absorbent cotton or the like. In particular, (A) a tool as shown in Fig. 2 is preferable because saliva does not soak into the medium in the collecting tool and can be easily transferred to the sensor chip 1. In the saliva-collecting tool 3 shown in Fig. 2, the medium for collecting saliva is polyamide villi. Since saliva does not soak into polyamide villi and is present on the surface of the polyamide villi, the saliva can be easily transferred to the sensor chip 1. As the polyamide, a so-called Nylon (registered trademark) is typically used.

Hereinafter, the stress measurement method of the present invention will be described. The stress measurement method of the present invention is a method using the above-described stress measurement kit, comprising the steps of:
(1) allowing the polysaccharide to act on collected saliva,
(2) developing the saliva, on which the polysaccharide has acted, in the electrode system;
(3) measuring a current generated in the electrode system, as a current value or an electric quantity, using the electrical measurement means; and
(4) converting the current value or the electric quantity measured by the electrical measurement means, into an amylase activity value or a stress level, using the conversion means.

The saliva that can be collected in the above-described step (1) is not limited to that of humans, and may be that of pets cared for in everyday life, such as dogs, cats, rabbits, and hamsters, or farm animals, such as pigs, horses, sheep, and cattle. With the recent pet boom, measurement of stress in pets is in demand. Furthermore, in the case where farm animals are raised in a stress-free environment through management using the stress measurement kit of the present invention, high-quality food can be obtained. Typically, an α-amylase activity in saliva of these animals tends to be lower than that of humans, and, thus, it can be said that the stress measurement kit of the present invention is also useful for measuring the stress level of these animals.

There is no particular limitation on the above-described step (1) as long as a polysaccharide can act on collected saliva. For example, in an embodiment in which a polysaccharide is immobilized on the working electrode 121 and in an embodiment in which a conjugate pad impregnated with a polysaccharide is disposed in a flow path that guides saliva from the mouth, from which saliva is collected, to the sensor chip 1, the polysaccharide can act on collected saliva by collecting the saliva in the sensor chip 1. It should be noted that, in this embodiment, the above-described step (2) is simultaneously performed.

Furthermore, in the embodiment in which the sensor body 2 is provided with a reagent chamber that contains a polysaccharide, and in which a flow path that guides the polysaccharide from the reagent chamber to the electrode system is provided in the sensor body 2, the above-described step (1) is realized by guiding the polysaccharide from the reagent chamber to the electrode system 12 and collecting saliva in the sensor chip 1 in a state where the sensor chip 1 is attached to the sensor body 2. It should be noted that, in this embodiment, the above-described step (2) is simultaneously performed.

Furthermore, in the embodiment in which absorbent cotton or the like of the saliva-collecting tool 3 (described later) is impregnated with a polysaccharide, the above-described step (1) can be realized simply by collecting saliva in the absorbent cotton or the like. In this embodiment, if the absorbent cotton or the like contains a sweetener or the like, a test subject can feel the sweetness when collecting saliva, and, thus, saliva can be stably collected. It can be said that this embodiment is preferable in particular in the case where a test subject is a child. The absorbent cotton or the like in which the saliva has been collected is accommodated in a provided container.

There is no particular limitation on the above-described step (2) as long as the saliva on which the polysaccharide has acted can be developed in the electrode system 12. As described above, in most embodiments of the present invention, the step (2) is performed simultaneously with the above-described step (1). It should be appreciated that, in an embodiment in which absorbent cotton or the like is impregnated with a polysaccharide, the step (2) is realized by transferring the polysaccharide from the absorbent cotton or the like to the sensor chip 1 in a provided container.

In the measurement in the above-described step (3), whether an item to be measured is a current value or an electric quantity depends on the type of the electrical measurement means 22.

As described above, in the above-described step (4), calculation can be performed using a calibration curve plotted in advance, and, thus, a detailed description thereof will not be provided herein. The results obtained through calculation by the conversion means 23 are displayed on a display means (not shown) in the sensor body 2. At that time, an amylase activity value itself may be displayed, or the amylase activity value may be converted into another parameter referred to as a stress level and then displayed. Furthermore, the item to be displayed is not limited to numerical values, and characters corresponding to the numerical values may be displayed. As such a character, a soothing character is preferable, and the Rilakkuma (registered trademark) is particularly preferable.

### Examples

### Example 1

A sensor chip as shown in Fig. 1 was prepared. More specifically, this was a sensor chip in which a working electrode 121 and a counter electrode 122 were formed on a polyester substrate 1. Here, the polyester substrate 1 was in the shape of a rectangle having a length of approximately 32 mm and a width of approximately 6 mm. Furthermore, the material of the working electrode 121 was graphite, and this graphite contained potassium hexacyanoferrate(III) as an electron mediator. On the other hand, the material of the counter electrode 122 was silver/silver chloride.

Furthermore, glucose dehydrogenase (GDH) and pyrroloquinoline quinone (PQQ) were immobilized on the working electrode 121. The immobilization was performed by applying an MES solution containing glucose dehydrogenase (GDH) and the pyrroloquinoline quinone (PQQ) to the working electrode 121 and then drying the solution.

Furthermore, soluble starch (manufactured by Nacalai Tesque, Inc.) was immobilized on the working electrode 121. More specifically, approximately 0.5 to 2 µl of soluble starch-containing MES solution (concentration: approximately 250 g/l) was applied thereon and then dried. In this manner, the sensor chip 1 of the present invention was produced.

### Example 2

The sensor chip 1 of the present invention was produced as in Example 1 except that cornstarch (manufactured by Nacalai Tesque, Inc.) was used instead of soluble starch.

### Example 3

The sensor chip 1 of the present invention was produced as in Example 1 except that glycogen (manufactured by Nacalai Tesque, Inc.) was used instead of soluble starch.

### Experimental Examples

α-Amylase solutions prepared so as to have different concentrations were developed in the electrode systems of the working electrode 121 and the counter electrode 122 in the sensor chips of Examples 1 to 3, and the current values generated were measured with a potentiostat. The results are shown in Fig. 3. According to Fig. 3, it was confirmed that sufficient current values were obtained in all measurements. It is clear that the obtained current values were much higher than those in the results presented in Fig. 21 of Patent Document 3.

### Industrial Applicability

The stress measurement kit of the present invention has a compact sensor body, and, thus, will be widely used as a portable stress measurement apparatus. Furthermore, sufficient sensitivity can be obtained, and, thus, the influence of errors is small, and amylase activity values and stress levels can be accurately measured.

## Claims

1. A stress measurement kit for measuring a stress level of a test subject, comprising:
(A) a sensor chip including an electrically insulating substrate, and an electrode system that is placed on the electrically insulating substrate and that includes at least a working electrode and a counter electrode, wherein glucose dehydrogenase (GDH) and an electron mediator are immobilized on the working electrode;
(B) a polysaccharide; and
(C) a sensor body including an insertion hole into which the sensor chip is inserted, an electrical measurement means that is electrically connected to the electrode system of the sensor chip in a state where the sensor chip is inserted into the insertion hole, and a conversion means by which a current value or an electric quantity measured by the electrical measurement means is converted into an amylase activity value.

2. The stress measurement kit of claim 1, wherein the polysaccharide is immobilized on the working electrode of the sensor chip.

3. The stress measurement kit of claim 1, wherein a coenzyme of glucose dehydrogenase is further immobilized on the working electrode.

4. The stress measurement kit of claim 3, wherein the coenzyme of glucose dehydrogenase is at least one selected from the group consisting of pyrroloquinoline quinone (PQQ), nicotinamide adenine dinucleotide (NAD), and nicotinamide adenine dinucleotide phosphate (NADP).

5. The stress measurement kit of claim 1, further comprising a saliva-collecting tool.

6. The stress measurement kit of claim 5, wherein the saliva-collecting tool includes a shaft member, a grip portion that is provided at one end of the shaft member, a villous support that is provided at another end of the shaft member, and polyamide villi that are supported by the villous support.

7. A stress measurement method for measuring a stress level of a test subject using a stress measurement kit,
wherein the stress measurement kit comprises:
(A) a sensor chip including an electrically insulating substrate, and an electrode system that is placed on the electrically insulating substrate and that includes at least a working electrode and a counter electrode, wherein glucose dehydrogenase (GDH) and an electron mediator are immobilized on the working electrode;
(B) a polysaccharide; and
(C) a sensor body including an insertion hole into which the sensor chip is inserted, an electrical measurement means that is electrically connected to the electrode system of the sensor chip in a state where the sensor chip is inserted into the insertion hole, and a conversion means by which a current value or an electric quantity measured by the electrical measurement means is converted into an amylase activity value or a stress level; and
the stress measurement method comprises the steps of:
(1) allowing the polysaccharide to act on collected saliva;
(2) developing the saliva, on which the polysaccharide has acted, in the electrode system;
(3) measuring a current generated in the electrode system, as a current value or an electric quantity, using the electrical measurement means; and
(4) converting the current value or the electric quantity measured by the electrical measurement means, into an amylase activity value or a stress level, using the conversion means.

8. The stress measurement method of claim 7,
wherein the polysaccharide is immobilized on the working electrode of the sensor chip, and
the steps (1) and (2) are simultaneously performed.

9. The stress measurement method of claim 8, wherein the step (1) is performed by transferring saliva collected by a saliva-collecting tool, to the sensor chip.

10. The stress measurement method of claim 9, wherein the saliva-collecting tool includes a shaft member, a grip portion that is provided at one end of the shaft member, a villous support that is provided at another end of the shaft member, and polyamide villi that are supported by the villous support.
